# EUROPEAN PATENT SPECIFICATION

(11) **EP 0 558 529 B1**
(45) Date of publication and mention of the grant of the patent: **10.01.1996**
(21) Application number: 91919614.7
(22) Date of filing: 18.10.1991
(51) Int. Cl.: A61K 39/395

(54) **PHARMACEUTICAL PREPARATION FOR THE TREATMENT OF PROLONGED COAGULATION TIME**
PHARMAZEUTISCHE ZUBEREITUNG ZUR BEHANDLUNG VERLÄNGERTER GERINNUNGSZEIT
PREPARATION PHARMACEUTIQUE POUR LE TRAITEMENT D'UN TEMPS DE COAGULATION PROLONGE

(30) Priority: 31.10.1990 DK 2614/90
(43) Date of publication of application: 08.09.1993
(73) Proprietor: NOVO NORDISK A/S, DK-2880 Bagsvaerd (DK)
(72) Inventor: NORDFANG, Ole, DK-3400 Hilleroed (DK); HEDNER, Ulla, S-21 620 Malmö (SE); MOLLER, Niels, Peter, Hundahl, DK-2800 Lyngby (DK)
(86) International application number: DK9100317
(87) International publication number: WO9207584

(56) References cited:
- EP-A- 0 300 988
- WO-A-89/08666
- THROMBOSIS RESEARCH, vol. 64, 1991; D.J. WELSCH et al., pp. 213-222/
- THROMBOSIS RESEARCH, suppl XIV, 1991; A.K. LINDAHL et al., pp. 39-48/
- DIALOG INFORMATION SERVICES, File 155, Medline 66-92; A.K. LINDAHL et al., AN 07885158/
- NATL. LIBRARY OF MEDICINE, File Medline; S. MASSONNET-CASTEL et al., AN 86221927/
- BIOCHEMISTRY, vol. 29, no. 33, 1990; G.J. BROZE, Jr. et al., pp. 7539-7546/
- BLOOD, vol. 73, no. 2, 1989; S.I. RAPAPORT, pp. 359-365/
- DIALOG INFORMATION SERVICES, File 155, Medline 66-92; P.M. SANDSET et al., AN 06677231/

## Description

### FIELD OF INVENTION

The present invention relates to agents that block EPI activity and the use of these agents to produce preparations that will affect the haemostatic balance.

### BACKGROUND OF THE INVENTION

Coagulation is a complex process involving many protein factors. The coagulation process can be initiated via the intrinsic pathway through contact with a foreign surface or via the extrinsic pathway through contact with damaged tissue. By extrinsic activation the circulating blood comes in contact with tissue factor (TF). TF is a cofactor for coagulation factor VII (FVII) and the TF-FVII/TF-FVIIa complex activates FX and FIX. Due to the activation of FIX, "intrinsic coagulation" is also influenced by TF activation and an increasing amount of evidence indicate that TF-FVII activation is most important for the initiation of the coagulation reaction (Österud and Rapaport, Proc Natl. Acad Sci USA **74**, p 5260, 1977). The coagulation factors of the extrinsic pathway are inhibited by EPI (Extrinsic Pathway Inhibitor) also called LACI (Broze and Miletich, Blood **69**, p 150, 1987). EPI is a Kunitz type protease inhibitor which binds and inhibits FXa. The EPI-FXa complex inhibits FVIIa-TF (Rapaport, Blood **73**, p 359, 1989). The significance of EPI for the coagulation reaction has not yet been established since only unphysiological high concentrations affects conventional coagulation assays (Broze et al., Biochemistry **29**, p 7539, 1990). Therefore, EPI activity is measured in sophisticated assays where EPI is allowed to bind FXa and the EPI-FXa is added to TF-FVII reactants that may activate FIX or FX (Bajaj et al., J Clin Invest **79**, p 1974, 1987; Sandset et al., Thromb Res **47**, p 389, 1987).
In normal haemostasis the coagulation factors are in balance with each other. However, in some circumstances excessive bleeding is observed. Examples are: haemophilia caused by lack of FVIII or FIX, Idiopatic Thrombocytopenia (ITP) caused by reduced platelet counts. Also surgery often causes excessive bleeding. Examples of agents that can be used for the prevention of bleeding are: FIX, FVIII, FVIIa, aprotinin and tranexamic acid.

S. Massonet-Castel et al., Haemostasis 16, 1986, pp. 139-146, describe the neutralization of heparin by protamine sulfate to reverse the anti-IIa and anti-Xa activity of heparin, thereby correcting the thrombin time and the activated partial thromboplastin time. There is no indication that protamine sulfate might have any effect as an EPI inhibitor.

P.M. Sandset et al., Thrombosis Research 50, 1988, pp. 803-813, discuss the effect of heparin on EPI, noting that the increase in EPI activity caused by heparin is reduced by addition of polybrene and/or antibodies to antithrombin (anti-AT). Polybrene neutralizes heparin and anti-AT neutralizes antithrombin. Both substances are added to neutralize the antithrombin III/heparin in the EPI assay described. However, there is no indication that polybrene or anti-AT are EPI inhibitors.

### DISCLOSURE OF THE INVENTION

The present invention shows that agents which block EPI activity will also be able to reduce bleeding tendencies. This is surprising since EPI by itself has not yet been shown to affect the haemostatic balance. However, we have made the following new observations:
1. In coagulation assay plasma EPI is more active than EPI produced by recombinant technique (rEPI).
2. Coagulation induced by very dilute TF is dependent on plasma EPI and thus EPI affects the haemostatic balance.
3. Antibodies that block EPI activity (anti-EPI) shorten the coagulation time of normal plasma.
4. Anti-EPI shortens the coagulation time of haemophilia plasma.

These observations indicate that blocking of EPI will reduce bleeding tendencies.

Previously it has only been possible to accelerate the coagulation process in normal plasma by adding a coagulation initiator (tissue factor, kaolin, etc.) or an active enzyme (thrombin, FXa, etc). Our experiments provide evidence that it is also possible to accelerate the coagulation process by blocking a plasma protein (EPI).

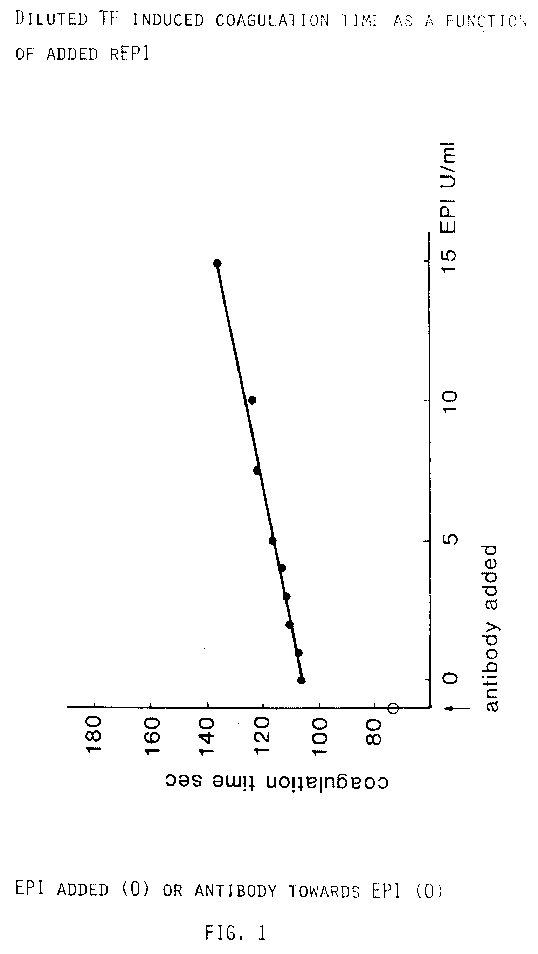

Intrinsic coagulation is usually measured in APTT assays where coagulation is initiated by Kaolin, phospholipids and Ca²⁺. High concentrations of EPI are needed to affect the APTT assay (Broze, Biochemistry **29**,, p 7539, 1990). Extrinsic coagulation is measured in PT assays where coagulation is initiated by tissue factor (brain extract) and Ca²⁺. In the standard assay undiluted tissue factor is used and addition of EPI has only little effect on the coagulation times observed (see example 2). A much more physiological assay is obtained when the tissue factor solution is diluted considerably. Then the coagulation time becomes dependent on extrinsic as well as intrinsic coagulation factors (Brinkhous et al. Proc Natl Acad Sci USA **86**, p 1382, 1989). Fig. 1 shows that addition of rEPI increases the coagulation time by approximately two seconds for each EPI unit added. Surprisingly addition of antibody towards EPI (removal of one EPI unit) shorten the coagulation time by 30 seconds. Control experiments were performed to ensure that the effect was really due to inhibition of EPI.

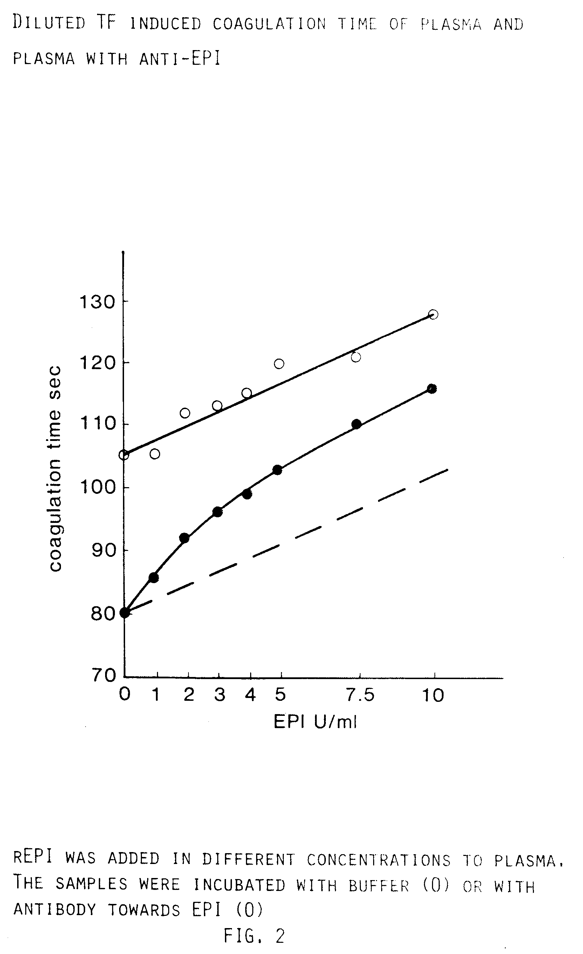

The following results were found:
A. Addition of excess antibody did not further shorten the coagulation time.
B. The titer of the antibody in EPI activity inhibition assay corresponds to the titer observed in coagulation time shortening assay.
C. The antibody was affinity purified by pure rEPI coupled to Sepharose®. The resulting IgG preparation showed on a mg basis a 10 fold increase in titer towards coagulation time shortening as well as towards EPI activity.
D. When antibody was added in an amount just sufficient to shorten the coagulation time then addition of rEPI had a more pronounced effect on the coagulation time increase compared with addition to plasma without antibody (Fig. 2).
E. EPI deficient plasma was prepared from normal plasma by immunoabsorption. This plasma had a reduced coagulation time compared with normal plasma. Addition of antibody in the coagulation analysis did not further shorten the coagulation time of the deficient plasma.

Antibodies that block EPI activity have been described in the literature (Novotny et al. Blood **72**, p 2020, 1988). However, inhibition of plasma EPI by an antibody has not been described and it has not been observed that such an antibody may have a significant direct effect on coagulation and consequently on the haemostatic balance.

To measure EPI, Novotny et al. use a complicated 3 stage coagulation assay. EPI sample is first incubated with TF, FVIIa, FX and Ca²⁺ is added. Then FX is added and after 1 min. incubation FX deficient plasma and rabbit brain cephalin are added. In this assay EPI inhibits the TF activity and it has been shown that anti EPI antibody inhibits the effect of EPI.

In a one stage coagulation assay a mixture of Ca²⁺ and TF are added directly to plasma and the coagulation time is recorded.

In such an assay Broze et al. (Biochemistry **29**, p 7543, 1990) found it necessary to add 2.5 µg EPI/ml (50 U/ml) to obtain just 50% reduction in apparent TF activity.

Surprisingly we find that adding anti-EPI to plasma (removing 1 U of EPI) significantly affect the coagulation time when coagulation was initiated directly by dilute TF. This coagulation assay mimics the physiological situation since it is the only type of coagulation assay that is dependent on the presence of both FVII, FVIII and FIX all factors that are necessary to obtain normal haemostasis.

Furthermore, plasma EPI has been purified (Novotny et al. J Biol Chem **264**, p 18832, 1989). However, it has not been observed that the plasma EPI has a significant effect on coagulation, at least not to such a degree that the inhibition of EPI would affect the haemostatic balance. Thus it has been stressed that documentation for the psysiologic importance of EPI remains to be provided (Broze, Biochemistry **29**, p 7539, 1990). Our observation, that plasma EPI significantly affects the coagulation time of plasma, provides evidence that EPI has an important role for the haemostatic balance. Furthermore, our experiments provide evidence that blocking of EPI will be able to reduce bleeding tendencies.

### SUMMARY OF THE INVENTION

In its first aspect the present invention is, thus, related to a pharmaceutical preparation for the treatment of patients with prolonged coagulation time, the preparation containing, as an active component, an EPI inhibitor characterized by inhibiting EPI in the "Bethesda assay" described herein and reducing the coagulation time of plasma in the coagulation assay described herein, with the exception of heparin binding protein (HBP).

In a second aspect the invention relates to the use of an EPI inhibitor for the production of a pharmaceutical preparation for the treatment of patients with prolonged coagulation time, said EPI inhibitor being characterized by inhibiting EPI in the "Bethesda assay" described herein and reducing the coagulation time of plasma in the coagulation assay described herein.

### EXPERIMENTAL PART

Preparation of anti-EPI antibodies: Recombinant human EPI (rEPI) was obtained from transfected BHK cells as described by Pedersen et al. (J Biol Chem, **265**, p 6786-6793, 1990). rEPI was purified by heparin affinity chromatography, ion exchange and reversed phase chromatography (Nordfang et al., Biotech Plasma Prot p 98, 1990). rEPI obtained in this way was pure judged from SDS-PAGE. Rabbits were immunized on day 0, 14, 35 followed by 21 days intervals. Each immunization was with 0.1 mg of rEPI in adjuvans. The first immunization was with Freunds complete adjuvants while the next immunizations were with Freunds incomplete adjuvans. The antisera obtained were tested for inhibition of EPI activity in EPI activity assay and the inhibition was quantitated like FVIII inhibiting antibodies in Bethesda assay: equal volumes of diluted antiserum and EPI (1 U/ml) were incubated for 2 hours at 37°C. EPI activity was measured, and the dilution of antiserum that inhibits the activity by 50% gives the titer. The rabbit antisera had inhibiting titers between 1000 and 4000 "Bethesda-like" units/ml towards both rEPI and human plasma EPI. Below 50 fold dilution serum from unimmunized rabbits did not influence the activity of the EPI sample (1 U/ml). IgG was purified from the antisera by anion exchange chromatography. The IgG preparation with 8 mg IgG/ml contained 2000 "Bethesda-like" inhibiting units/ml towards human plasma EPI.

Assay for EPI activity: EPI was measured in a chromogenic microplate assay, modified after the method of Sandset et al., (Thromb Res **47**, P 389, 1989). Heat treated plasma pool was used as a standard. This standard is set to contain 1 U/ml of EPI activity. Standards and samples were diluted in buffer A (0.05 M tris/0.1 M NaCl/0.1 M Na-citrate/0.02% NaN₃/pH 8.0) containing 2 µg/ml polybrene and 0.2% bovine serum albumin. FVIIa/TF/FX/CaCl₂ combination reagent was prepared in buffer A and contained 1.6 ng/ FVIIa (Novo Nordisk A/S), human tissue factor diluted 60 fold (Hjort, Scand J Clin Lab Invest **9**, 1957), 50 ng/ml FX (Sigma) and 18 mM CaCl₂. The assay was performed in microplate strips at 37°C. 50 µl of samples and standards were pipetted into the strips and 100 µl combination reagent was added to each well. After 10 minutes incubation, 50 µl of FX (3.2 µg/ml) was added to each well and after another 10 minutes 25 µl of chromogenic substrate for FXa (S2222) was added 10 minutes after the addition of substrate. The reaction was stopped by addition of 50 µl 1.0 M citric acid pH 3.0. The microplate was read at 405 nm.

Coagulation assay: Coagulation activity was measured using an ACL coagulation apparatus. 20 µl of antibody solution or EPI solution was incubated with 200 µl plasma for 15 minutes at room temperature. After preheating to 37°C the ACL mixes 75 µl of plasma sample with 75 µl of diluted TF in 20 mM CaCl₂, 50 mM NaCl, 17 mM imidazole, 33 µg/ml BSA, pH 7.4.

### EXAMPLE 1

Coagulation assay was performed as described above using 20,000 fold dilution of tissue factor. Table 1 shows the effect of adding anti-EPI to 10 individual donor samples, 7 haemophilia A samples and 1 haemophilia B sample. It appears that a considerable shortening of coagulation time was observed, especially for the haemophilia samples. These data reflect the tentative greater importance of a powerfull extrinsic, FVII dependent pathway in the case of an impaired intrinsic coagulation pathway (haemophilia A and B).

**Table 1**

| Effect of anti-EPI on the coagulation time of individual plasma samples. | | | |
|---|---|---|---|
| | donors (n=10) | haem A (n=7) | haem B (n=1) |
| Coagulation time without Ab range | 102 91-113 | 131 104-165 | 165 |
| Coagulation time with anti-EPI range | 77 71-87 | 92 82-113 | 116 |
| Reduction, sec. range | 26 11-39 | 39 22-63 | 49 |

### EXAMPLE 2

Coagulation assay was performed with a normal plasma pool using different dilutions of TF. Table 2 shows that the more TF is diluted, the more significant is the effect of adding anti EPI to plasma. This illustrates that the longer the coagulation time, the more effective will anti-EPI be in shortening the coagulation time.

**Table 2**

| Effect of adding anti-EPI or EPI as a function of coagulation time. | | | | | |
|---|---|---|---|---|---|
| | Coagulation time in seconds | | | | |
| Dilution of TF | Normal plasma | Normal plasma with anti-EPI | % of NP | NP with 16 U/ml of rEPI add. | %of NP |
| 60 | 16.6 | 16.1 | 97 | 17.6 | 106 |
| 300 | 28.1 | 24.5 | 87 | 31.4 | 112 |
| 1,500 | 49.0 | 38.9 | 79 | 59.0 | 120 |
| 7,500 | 92.0 | 64.8 | 70 | 123 | 134 |
| 15,000 | 121 | 80.5 | 67 | >165 | >136 |
| 21,000 | 143 | 91.3 | 64 | >165 | > |
| 30,000 | >165 | 102 | <62 | >165 | > |
| 45,000 | >165 | 117 | < | >165 | > |

### EXAMPLE 3

Citrate plasma was drawn from a patient after Cardiopulmonary bypass surgery (CPB). In this procedure the patient is anti-coagulated with large doses of heparin. After operation the heparin is neutralized by injection of protamine sulphate. Measured by a chromogenic FXa inhibition analysis the plasma sample contained no heparin. In Sandset EPI assay the sample contained 2 U/ml of EPI activity. Table 3 shows the dilute TF coagulation time of this plasma before and after addition of anti-EPI. It appears that the coagulation time is prolonged and that anti-EPI can shorten the coagulation time. Thus increased EPI levels may be one of the reasons why CPB patients bleed during and after the operation.

**Table 3**

| Effect of anti-EPI on the coagulation time of plasma from a CPB patient. | | |
|---|---|---|
| | Donor pool plasma | CPB patient plasma |
| Coagulation time without antibody | 100 | >165 |
| Coagulation time with anti-EPI | 70 | 82 |

### EXAMPLE 4

Heparin binding protein (HBP) WO89/08666 is a protein from neutrophil granula that shows high homology to elastase. HBP is not an enzyme by itself, but seems to bind enzyme inhibitors. When tested in EPI assay the titer of HBP towards EPI is 10 "Bethesda like" units/mg. HBP also blocks the inhibition of FXa by EPI with a corresponding titer. When added to normal plasma in a concentration of 150 µg/ml, the dilute TF induced coagulation time was shortened (Table 4).

**Table 4**

| Effect of HBP on the coagulation time of normal plasma: | |
|---|---|
| | Coagulation time, sec. |
| Normal plasma | 98 |
| Normal plasma with anti-EPI | 70 |
| Normal plasma with HBP | 81 |

## Claims (Claims for the following Contracting State(s): AT, BE, CH, DE, DK, FR, GB, IT, LI, LU, NL, SE)

1. A pharmaceutical preparation for the treatment of patients with prolonged coagulation time, the preparation containing, as an active component, an EPI (Extrinsic Pathway Inhibitor) inhibitor characterized by inhibiting EPI in the "Bethesda assay" described herein and reducing the coagulation time of plasma in the coagulation assay described herein, with the exception of heparin binding protein (HBP).

2. A pharmaceutical preparation according to claim 1, wherein the coagulation malfunction is due to haemophilia A or B.

3. A pharmaceutical preparation according to claim 1, wherein the coagulation malfunction is due to Idiopatic Thrombocytopenia (ITP), surgery procedures or DIC (disseminated intravascular coagulation).

4. Use of an EPI inhibitor for the production of a pharmaceutical preparation for the treatment of patients with prolonged coagulation time, said EPI inhibitor being characterized by inhibiting EPI in the "Bethesda assay" described herein and reducing the coagulation time of plasma in the coagulation assay described herein.

5. Use according to claim 4, wherein the coagulation malfunction is due to haemophilia A or B.

6. Use according to claim 4, wherein the coagulation malfuntion is due to Idiopatic Thrombocytopenia (ITP), surgery procedures or DIC.

## Claims (Claims for the following Contracting State(s): ES, GR)

1. Use of an EPI (Extrinsic Pathway Inhibitor) inhibitor for the production of a pharmaceutical preparation for the treatment of patients with prolonged coagulation time, said EPI inhibitor being characterized by inhibiting EPI in the "Bethesda assay" described herein and reducing the coagulation time of plasma in the coagulation assay described herein.

2. Use according to claim 1, wherein the coagulation malfunction is due to haemophilia A or B.

3. Use according to claim 1, wherein the coagulation malfunction is due to Idiopatic Thrombocytopenia (ITP), surgery procedures or DIC.

## Patentansprüche (Patentansprüche für folgende(n) Vertragsstaat(en): AT, BE, CH, DE, DK, FR, GB, IT, LI, LU, NL, SE)

1. Eine pharmazeutische Zubereitung zur Behandlung von Patienten mit verlängerter Gerinnungszeit, wobei die Zubereitung, als einen aktiven Bestandteil, einen EPI(Extrinsic Pathway Inhibitor)-Inhibitor enthält, gekennzeichnet durch die Inhibition von EPI im hierin beschriebenen "Bethesda-Test" und die Verringerung der Gerinnungszeit von Plasma im hierin beschriebenen Gerinnungstest, mit der Ausnahme von Heparin-Bindungsprotein (HBP).

2. Eine pharmazeutische Zubereitung nach Anspruch 1, wobei die Gerinnungsfehlfunktion auf Hämophilie A oder B beruht.

3. Eine pharmazeutische Zubereitung nach Anspruch 1, wobei die Gerinnungsfehlfunktion auf idiopatischer Thrombozytopenie (ITP), chirurgischen Eingriffen oder DIC (dissiminierte intravaskuläre Gerinnung) beruht.

4. Verwendung eines EPI-Inhibitors zur Herstellung einer pharmazeutischen Zubereitung zur Behandlung von Patienten mit verlängerter Gerinnungszeit, wobei besagter EPI-Inhibitor gekennzeichnet ist durch die Inhibition von EPI im hierin beschriebenen "Bethesda-Test" und die Verringerung der Gerinnungszeit von Plasma im hierin beschriebenen Gerinnungstest.

5. Verwendung nach Anspruch 4, wobei die Gerinnungsfehlfunktion auf Hämophilie A oder B beruht.

6. Verwendung nach Anspruch 4, wobei die Gerinnungsfehlfunktion auf idiopatischer Thrombozytopenie (ITP), chirurgischen Eingriffen oder DIC beruht.

## Patentansprüche (Patentansprüche für folgende(n) Vertragsstaat(en): ES, GR)

1. Verwendung eines EPI(Extrinsic Pathway Inhibitor)-Inhibitors zur Herstellung einer pharmazeutischen Zubereitung zur Behandlung von Patienten mit verlängerter Gerinnungszeit, wobei besagter EPI-Inhibitor gekennzeichnet ist durch die Inhibition von EPI im hierin beschriebenen "Bethesda-Test" und die Verringerung der Gerinnungszeit von Plasma im hierin beschriebenen Gerinnungstest.

2. Verwendung nach Anspruch 1, wobei die Gerinnungsfehlfunktion auf Hämophilie A oder B beruht.

3. Verwendung nach Anspruch 1, wobei die Gerinnungsfehlfunktion auf idiopatischer Thrombozytopenie (ITP), chirurgischen Eingriffen oder DIC beruht.

## Revendications (Revendications pour l'(les) Etat(s) contractant(s) suivant(s): AT, BE, CH, DE, DK, FR, GB, IT, LI, LU, NL, SE)

1. Préparation pharmaceutique pour le traitement de patients dont le temps de coagulation est prolongé, la préparation contenant, en tant que principe actif, un inhibiteur EPI (inhibiteur de la voie extrinsèque), caractérisée par l'inhibition de l'EPI, dans "l'analyse Bethesda" décrite ici, et réduisant le temps de coagulation du plasma dans l'analyse de coagulation décrite ici, à l'exception de la protéine liant l'héparine (HBP).

2. Préparation pharmaceutique selon la revendication 1, dans laquelle le dysfonctionnement de la coagulation est dû à l'hémophilie A ou B.

3. Préparation pharmaceutique selon la revendication 1, dans laquelle le dysfonctionnement de la coagulation est dû à la thrombocytopénie idiopathique (ITP), à des interventions chirurgicales ou à une DIC (coagulation intravasculaire disséminée).

4. Utilisation d'un inhibiteur EPI pour la production d'une préparation pharmaceutique destinée au traitement de patients présentant un temps de coagulation prolongé, l'inhibiteur EPI étant caractérisé par l'inhibition de l'EPI dans "l'analyse Bethesda" décrite ici et réduisant le temps de coagulation du plasma dans l'analyse de coagulation décrite ici.

5. Utilisation selon la revendication 4, dans laquelle le dysfonctionnement de la coagulation est dû à l'hémophilie A ou B.

6. Utilisation selon la revendication 4, dans laquelle le dysfonctionnement de la coagulation est dû à la thrombocytopénie idiopathique (ITP), à des interventions chirurgicales ou à la DIC (coagulation intravasculaire disséminée).

## Revendications (Revendications pour l'(les) Etat(s) contractant(s) suivant(s): ES, GR)

1. Utilisation d'un inhibiteur EPI (inhibiteur de la voie extrinsèque) pour la production d'une préparation pharmaceutique destinée au traitement de patients présentant un temps de coagulation prolongé, cet inhibiteur EPI étant caractérisé par l'inhibition de l'EPI dans "l'analyse Bethesda" décrite ici et réduisant le temps de coagulation du plasma dans l'analyse de coagulation décrite ici.

2. Utilisation selon la revendication 1, dans laquelle le dysfonctionnement de la coagulation est dû à l'hémophilie A ou B.

3. Utilisation selon la revendication 1, dans laquelle le dysfonctionnement de la coagulation est dû à une thrombocytopénie idiopathique (ITP), à des interventions chirurgicales ou à une DIC (coagulation intravasculaire disséminée).
